# EUROPÄISCHE PATENTSCHRIFT

(11) **EP 1 054 974 B1**
(45) Veröffentlichungstag und Bekanntmachung des Hinweises auf die Patenterteilung: **28.03.2007**
(21) Anmeldenummer: 99910229.6
(22) Anmeldetag: 18.02.1999
(51) Int. Cl.: C12N 15/53, C12P 7/42, C12P 7/62, C12P 11/00, C12P 13/02

(54) **VERFAHREN ZUR HERSTELLUNG VON TRIFLUOR-3(R)-HYDROXYBUTTERSÄUREDERIVATEN**
METHOD FOR PRODUCING TRIFLUORO-3(R)-HYDROXYBUTYRIC ACID DERIVATIVES
PROCEDE POUR LA PREPARATION DE DERIVES D'ACIDE TRIFLUOR-3(R)-HYDROXYBUTYRIQUE

(30) Priorität: 18.02.1998 CH 38898
(43) Veröffentlichungstag der Anmeldung: 29.11.2000
(73) Patentinhaber: Lonza AG, 3930 Visp (CH)
(72) Erfinder: PETERSEN, Michael, CH-3930 Visp (CH); BIRCH, Olwen, CH-3930 Visp (CH); SHIMIZU, Sakayu, Kyoto 606-8502 (JP); KIENER, Andreas, CH-3930 Visp (CH); HISCHIER, Marie-Luise, CH-3930 Visp (CH); THÖNI, Susanne, CH-3904 Naters (CH)
(74) Vertreter: Reiss, Gilles François
(86) Internationale Anmeldenummer: PCT/EP1999/001017
(87) Internationale Veröffentlichungsnummer: WO 1999/042590

(56) Entgegenhaltungen:
- EP-A- 0 645 453
- WO-A-93/18138
- KITA: "cloning of the aldehyde reductase gene from a red yeast, Sporobolomyces salmonicolor, and characterization of the gene and its product" APPLIED AND ENVIRONMENTAL MICROBIOLOGY, Bd. 62, Nr. 7, Juli 1996, Seiten 2303-2310, XP002105940
- EHRLER ET AL: "Notiz über microbiologische Umsetzungen mit Halobacterium halobium: Reduktion von 3-Oxobutansäure-ethylester und Hydrolyse von 3-Hydroxybutansäure-ethylester. Cooperative Effekte von Reduktase und Hydrolase" HELVETICA CHIMICA ACTA, Bd. 72, 1989, Seiten 793--799, XP002008201

## Beschreibung

Die Erfindung betrifft ein neues biotechnologisches Verfahren zur Herstellung von Trifluor-3(R)-hydroxybuttersäurederivaten der allgemeinen Formel 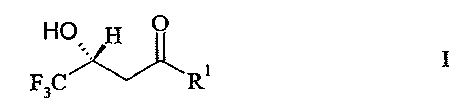 Trifluor-3(R)-hydroxybuttersäurederivate wie 4,4,4-Trifluor-3(R)-hydroxybuttersäureethylester sind wichtige Zwischenprodukte zur Herstellung von Pharmazeutika wie beispielsweise zur Herstellung von Befloxatone, einem Monoaminoxidase-A-Inhibitor (EP-A 0 736 606).

Zur Herstellung von 4,4,4-Trifluor-3(R)-hydroxybuttersäureester sind bereits mehrere biotechnologisches Verfahren bekannt.
Guerrero, A. & Raja, E (Bioorganic Chemistry Letters 1(12), 675-678) beschreiben ein mikrobiologisches Verfahren zur Herstellung von 4,4,4-Trifluor-3(R)-hydroxybuttersäureethylester ausgehend von dem entsprechenden Racemat mittels *Saccharomyces cerevisae.* Dabei wird das gewünschte Produkt in schlechter Enantiomeren-Reinheit erhalten.
Die EP-A 0 736 606 beschreibt ein biotechnologisches Verfahren zur Herstellung von 4,4,4-Trifluor-3(R)-hydroxybuttersäureethylester, ausgehend von 4,4,4-Trifluor-3-hydroxybuttersäureethylester mittels der Lipase Novozym 435. Nachteilig bei diesem Verfahren ist die mässige Ausbeute an dem gewünschten Produkt.
Die EP-A 0 577 446 umfasst ein biotechnologisches Verfahren zur Herstellung von optisch aktivem 4,4,4-Trifluor-3-hydroxybuttersäureethylester, ausgehend von dem entsprechenden racemischen Ester mittels Lipasen. Gemäss dieses Verfahrens wird das Produkt in geringer Ausbeute und in schlechter optischer Reinheit erhalten.
Die WO93/18138 A beschreibt die Herstellung der entsprechenden 3(R)-Hydroxyverbindung aus 4,4,4-Trifluoro-3-ketobutanoatethylester mittels einer Reductase aus *C. parapsilosis* in immer noch ungenügender optischer Reinheit.
Die WO 89/02 470 beschreibt ein Verfahren zur Herstellung von 4,4,4-Trifluor-3(R)-hydroxybuttersäureethylester, ausgehend von racemischem 4,4,4-Trifluor-3-acyloxybuttersäureethylester mittels hydrolytischen Enzymen. Dabei wird jedoch das entsprechende Produkt nicht in enantiomerenreiner Form erhalten.
Kita et. al., 1996, Appl.Environm. Microbiol. 62, 2303-2310 und darauf aufbauend Kataoka, M. et al., 1998, Bioscience Biotechnol. Biochem. 62:167-169 beschreiben die Produktion chiralen 4-Chloro-3(R)-hydroxy-butanoats ausgehend vom 4-Chloro-3-oxobutanoat mittels einer Kombination aus einer *Sporobolomyces salmonicolor* Aldehydereductase und einer *Bacillus megaterium* Glucosedehydrogenase für die NADPHH+ Kofactorregeneration, in E.coli. Die optische Reinheit des so erhaltenen Produktes war wiederum nicht ausreichend.

Aufgabe der vorliegenden Erfindung war ein biotechnologisches Verfahren zur Herstellung von 4,4,4-Trifluor-3(R)-hydroxybuttersäurederivaten zur Verfügung zu stellen, mit welchem das gewünschte Produkt im wesentlichen optisch rein und mit guter Ausbeute isoliert werden kann.

Diese Aufgabe wird mit dem Verfahren nach Anspruch 1 gelöst.

Erfindungsgemäss wird das Verfahren derart durchgeführt, dass man ein Trifluoracetessigsäurederivat der allgemeinen Formel 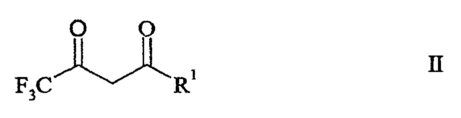 worin
R¹ = -OR², worin R² Wasserstoff, C₁₋₁₀-Alkyl, gesättigt oder ungesättigt, C₃₋₈Cycloalkyl, Aryl, Alkoxyalkyl oder Alkoxyalkoxyalkyl ist, oder
   -NR³R⁴, worin R³ und R⁴ gleich oder verschieden sind und für Wasserstoff, C₁₋₁₀-Alkyl, C₁₋₁₀-Alkenyl, C₃₋₈-Cycloalkyl oder Aryl stehen, oder
   -SR⁵, worin R⁵ Wasserstoff, C₁₋₁₀-Alkyl, C₁₋₁₀-Alkenyl, Aryl oder C₃₋₈-Cycloalkyl ist,
mittels Mikroorganismen, die befähigt sind eine Carbonylfunktion zu reduzieren oder mittels einem zellfreien Enzymextrakt dieser Mikroorganismen, in die Verbindung der allgemeinen Formel 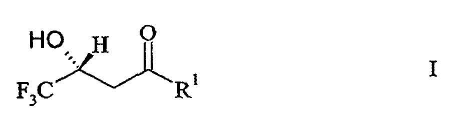 worin R¹ die genannte Bedeutung hat, überführt.

Als C₁₋₁₀-Alkyl kann im folgenden eine verzweigte oder unverzweigte primäre, sekundäre oder tertiäre aliphatische Gruppe wie Methyl, Ethyl, Propyl, Isopropyl, Butyl, Isobutyl, sec-Butyl, tert-Butyl, Pentyl, Isopentyl, sec-Pentyl, Hexyl, Heptyl, Octyl, Nonyl oder Decyl verwendet werden. Vorzugsweise bedeutet C₁₋₁₀-Alkyl, Ethyl, Propyl, Isopropyl oder Hexyl.

Als C₁₋₁₀-Alkenyl können beispielsweise Ethenyl, Propenyl, Allyl und Butenyl verwendet werden. Vorzugsweise wird Allyl verwendet.

Aryl bedeutet bevorzugt substituiertes oder unsubstituiertes Benzyl, Phenyl oder Naphtyl. Als substituiertes Benzyl kann beispielsweise halogeniertes Benzyl wie Chlor- oder Brombenzyl verwendet werden. Vorzugsweise wird unsubstituiertes Benzyl eingesetzt.

C₃₋₈-Cycloalkyl bedeutet bevorzugt Cyclopropyl, Cyclobutyl, Cyclopentyl, Cyclohexyl, Cycloheptyl oder Cyclooctyl, vorzugsweise Cyclohexyl.

Alkoxyalkyl bedeutet bevorzugt C₁₋₆-Alkoxyethyl wie Methoxyethyl und Ethoxyethyl, besonders bevorzugt Ethoxyethyl.

Alkoxyalkoxyalkyl bedeutet bevorzugt 2-(2-C₁₋₆-Alkoxy-ethoxy)-ethyl wie 2-(2-Methoxy-ethoxy)-ethyl und 2-(2-Ethoxy-ethoxy)ethyl, wobei letzteres besonders bevorzugt eingesetzt wird.

Bevorzugte Edukte sind demnach Trifluoracetessigsäureethyl-, Trifluoracetessigsäurepropyl-, Trifluoracetessigsäureisopropyl- und Trifluoracetessigsäurehexylester, Trifluoracetessigsäurecyclohexylester, Trifluoracetessigsäurebenzylester, Trifluoracetessigsäureethoxyethylester, und Trifluoracetessigsäureethoxyethoxyethylester.

Zweckmäßige Mikroorganismen, die befähigt sind, eine Carbonylfunktion zu reduzieren, sind beispielsweise Mikroorganismen, die ein exprimierbares Gen für ein Enzym enthalten, das befähigt ist eine Carbonylfunktion zu reduzieren, beispielsweise ein Enzym mit Reduktase-Aktivität, insbesondere ein Gen für eine Aldehydreduktase, eine Alkoholdehydrogenase oder eine Ketonreduktase. Die Enzyme, die befähigt sind, eine Carbonylfunktion zu reduzieren, können NADPHHH (β-Nikotinsäureamid-adenindinucleotidphosphat)-abhängig oder von anderen Cofaktoren abhängig sein. Vorzugsweise kommen Mikroorganismen mit NADPHHH-abhängigen Reduktionssystemen zum Einsatz.

Zellfreie Enzymextrakte dieser Mikroorganismen können durch fachmännisch übliche Methoden, beispielsweise durch French-Press-, Ultraschall- oder Lysozym-Methode, erhalten werden.

Zweckmässig wird die Biotransformation mittels Mikroorganismen durchgeführt, die eine Aldehydreduktase, insbesondere eine NADPHHH-abhängige Aldehydreduktase, enthalten.

Mikroorganismen, die eine NADPHHH-abhängige Aldehydreduktase enthalten, wie Mikroorganismen der Spezies Sporobolomyces salmonicolor, sind bereits von Shimizu et al., 1990, Applied and Environmental Microbiology, 56(8), 2374 - 2377 und Kataoka, M. et al., Biochimica et Biophysica Acta, 1122, 57-62 (1992), beschrieben. Diese Mikroorganismen können zum einen selbst für das erfindungsgemässe Verfahren eingesetzt werden, zum anderen als Ausgangsmaterial für die Konstruktion von Plasmiden und weiteren geeigneten Mikroorganismen dienen.

Zweckmässig werden für die Biotransformation rekombinante Mikroorganismen eingesetzt, die mit einem Gen codierend für ein Enzym, das befähigt ist eine Carbonylfunktion zu reduzieren, transformiert sind. Mikroorganismen, die mit einem solchen Gen transformiert sein können, sind beispielsweise Mikroorganismen der Gattung Escherichia, insbesondere der Spezies Escherichia coli, beispielsweise Escherichia coli JM109, Escherichia coli DH5 und Escherichia coli HB101.

Bevorzugt befindet sich das Gen mit der Reduktase-Aktivität, beispielsweise eine Aldehyd-Reduktase, auf einem zur Transformation geeigneten Vektor, beispielsweis einem Plasmid, zweckmäßig zusammen mit einem zur Expression des Gens geeigneten Promotor wie dem tac-Promotor (P_{tac}).

Sofern die verwendeten Mikroorgansimen NADPHHH-abhängige Enzyme enthalten, wird die Biotransformation zweckmäßig in Gegenwart von NADPHHH durchgeführt. Das NADPHHH wird entweder direkt in den erforderlichen Mengen zugesetzt oder in situ hergestellt. Vorteilhaft wird das NADPHHH in situ hergestellt. Zu diesem Zweck wird die Biotransformation zweckmäßig in Gegenwart eines NADPHHH-Generators oder Regenerators durchgeführt, d.h. eines Enzyms, das die Bildung von NADPHHH aus dessen oxidierter Form, NADPHH⁺, katalysiert. Zweckmäßig wird als NADPHHH-Generator oder -Regenerator eine Glucosedehydrogenase eingesetzt, beispielsweise Glucosedehydrogenase aus Bacillus megaterium.

Zur Generation von NADPHHH bei der Biotransformation wird diese zweckmäßig in Gegenwart eines Mikroorganismus durchgeführt, der den NADPHHH-Generator exprimiert. Insbesondere werden hierzu rekombinante Mikroorganismen verwendet, die mit dem für den NADPHHH-Generator codierenden Gen transformiert sind. Das Gen für den NADPHHH-Generator befindet sich hierbei bevorzugt auf einem zur Transformation geeigneten Vektor, beispielsweis einem Plasmid, zweckmäßig zusammen mit einem zur Expression des Gens geeigneten Promotor wie dem tac-Promotor (P_{tac}).

Für die Herstellung der Trifluor-3(R)-hydroxybuttersäurederivate der allgemeinen Formel I mit einem ein zur' Reduktion einer Carbonylfunktion befähigtes, NADPHHH-abhängiges Enzym, beispielsweise eine NADPHHH-abhängige Aldehyd-Reduktase, enthaltenden Mikroorganismus in Gegenwart eines NADPHHH-Generators können verschiedene Mikroorganismen eingesetzt werden, von denen einer zur Reduktion der Carbonylfunktion und einer zur Bildung von NADPHHH befähigt ist. Vorteilhaft enthalten die erfindungsgemäß verwendeten, zur Reduktion einer Carbonylfunktion befähigten Mikroorgansimen aber bereits selbst ein für einen NADPHHH-Generator oder -Regenerator codierendes Gen, beispielsweise ein Gen codierend für eine Gtucosedehydrogenase

Vorteilhaft werden für die Biotransformation rekombinante Mikroorgansimen eingesetzt, die mit einem für ein NADPHHH-abhängiges Enzym, beispielsweise einem für eine NADPHHH-abhängige Aldehydreduktase codierenden Gen, und einem für einen NADPHHH-Generator oder -Regenerator, beispielsweise einem für eine Glucosedehydrogenase codierenden Gen, transformiert sind. In einer möglichen Ausführungsform befinden sich diese Gene zur Expression auf einem einzigen Plasmid. In einer weiteren Ausführungsform liegen diese Gene auf verschiedenen, miteinander kompatiblen Plasmiden vor.

Die Biotransformation kann also vorteilhaft mittels Mikroorganismen durchgeführt werden, die enthalten:
- mindestens einen Vektor, beispielsweise ein Plasmid, der ein Gen für ein zur Reduktion einer Carbonylfunktion befähigtes Enzym, beispielsweise ein Aldehydreduktase-Gen, enthält,
- mindestens zwei Vektoren, beispielsweise Plasmide, von denen der eine ein Gen für ein zur Reduktion einer Carbonylfunktion befähigtes Enzym, beispielsweise ein Aldehydreduktase-Gen, und der andere ein Gen für einen NADPHHH-Generator oder -Regenerator, beispielsweise ein Glucosedehydrogenase-Gen; enthält, oder
- mindestens einen Vektor, beispielsweise ein Plasmid, der sowohl ein Gen für ein zur Reduktion einer Carbonylfunktion befähigtes Enzym, beispielsweise ein Aldehydreduktase-Gen, als auch ein Gen für einen NADPHHH-Generator oder -Regenerator, beispielsweise ein Glucosedehydrogenase-Gen; enthält.

Vorzugsweise wird die Biotransformation mittels Mikroorganismen der Spezies E. coli JMI109 oder E. coli DH5, transformiert mit mindestens zwei Plasmiden enthaltend jeweils ein Aldehydreduktase- oder ein Glucosedehydrogenase-Gen, oder mittels Mikroorganismen der Spezies E coli HB101 oder E. coli DH5, transformiert mit mindestens einem Plasmid, weiches beide Gene, das Aldehydreduktase- und das Glucosedehydrogenase-Gen enthält, durchgeführt. Insbesondere wird die Biotransformation mit E. coli JM109 und E. coli DH5, enthaltend ein Aldehydreduktase- und ein Glucosedehydrogenase-Gen, durchgeführt. Selbstverständlich kann die Biotransformation auch mit verschiedenen Mikroorganismen, die jeweils nur eines der genannten Gene enthalten, durchgeführt werden.

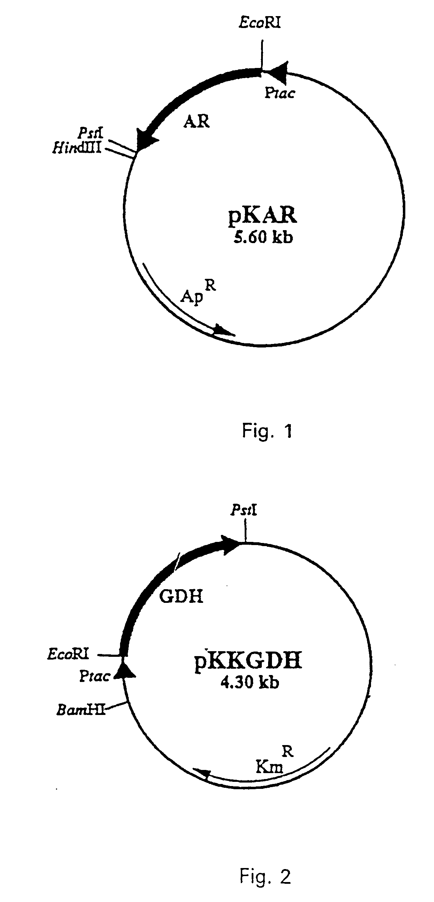

Fig. 1 zeigt die Struktur eines für die vorliegende Erfindung geeigneten Plasmids, pKAR, das das Gen für die NADPHHH-abhängige Aldehyd-Reduktase aus Sporobolomyces salmonicolor zusammen mit dem Promotor P_{tac} und einer Ampicillin (Ap)-Resistenz als Selektionsmarker enthält.

Fig. 2 zeigt die Struktur eines weiteren für die vorliegende Erfindung geeigneten Plasmids, pKKGDH, das das Gen für die Glucosedehydrogenase aus Bacillus megaterium zusammen mit dem Promotor P_{tac} und einer Kanamycin (Km)-Resistenz als Selektionsmarker enthält.

Der Mikroorganismus E. coli JM109, enthaltend das Plasmid pKAR mit einem Gen codierend für die NADPHHH-abhängige Aldehydreduktase aus Sporobolomyces salmonicolor und das Plasmid pKKGDH mit einem Gen codierend für die Glucosedehydrogenase aus Bacillus megaterium, wurde am 16.12.1997 bei der Deutschen Sammlung von Mikroorganismen und Zellkulturen GmbH (DSMZ), D-38124 Braunschweig, Mascheroderweg 1b, unter der Bezeichnung DSM 11902 gemäss Budapester Vertrag hinterlegt. Der Mikroorganismen E. coli DH5, enthaltend die Plasmide pKAR und pKKGDH, wurde am 7.12.1998 bei der zuvor beschriebenen Hinterlegungsstelle unter der Bezeichnung DSM 12566 gemäss Budapester Vertrag hinterlegt.

Die Expression der Gene kann abhängig vom Expressionssystem erfolgen. Bei den erfindungsgemäß bevorzugt verwendeten Expressionssystemen kann die Expression der Gene beispielsweise durch IPTG (Isopropylthiogalactosid) induziert werden, wenn als Mikroorganismus E. coli JM109 oder E. coli HB101 verwendet werden. Bei der Verwendung von E. coli DH5 ist die Induktion mit IPTG, wie fachmännisch bekannt, nicht notwendig.

Die Biotransformation kann nach üblichem Anzüchten der Zellen in einem einphasigen oder zweiphasigen System, vorzugsweise in einem zweiphasigen System, durchgeführt werden.

Als einphasiges System können fachmännisch übliche Puffer-Medien wie beispielsweise niedermolare Phosphatpuffer oder Tris-Puffer angewendet werden.

Als zweiphasiges System können die genannten fachmännisch üblichen Puffer-Medien zusammen mit einem für das Edukt löslichen organischen Lösungsmittel verwendet werden. Als organische Lösungsmittel sind beispielsweise Ester, Alkohole, halogenierte Kohlenwasserstoffe. Ether, aliphatische C₅₋₁₂-Kohlenwasserstoffe oder aromatische Kohlenwasserstoffe geeignet. Als Ester können Essigsäureester wie Essigsäuremethyl-, Essigsäureethyl-, Essigsäurepropyl- und Essigsäurebutylester verwendet werden. Als Alkohole können C₄₋₁₀-Alkohole wie Hexanol, Heptanol und Octanol verwendet werden. Als aromatische Kohlenwasserstoffe können beispielsweise Benzol, Toluol und Xylol verwendet werden. Als halogenierte Kohlenwasserstoffe können beispielsweise Chloroform und Dichlormethan verwendet werden. Als Ether können beispielsweise Diethylether, Tetrahydrofuran, Methyltert-butylether und Dibutylether verwendet werden. Als aliphatische C₅₋₁₂-Kohlenwasserstoffe sind beispielsweise Pentan, Hexan, Heptan, Octan, Nonan und Decan geeignet.

Geeignet ist ebenfalls ein zweiphasiges System in welchem die zweite Phase aus dem Edukt und / oder aus dem Produkt besteht. Um die Löslichkeit des Eduktes zu erhöhen, können Cosolvenzien eingesetzt werden. Als Cosolvenzien können entweder niedermolekulare aliphatische Alkohole wie beispielsweise Methanol, Ethanol, Propanol, Isopropanol, tert-Butanoi oder inerte Lösungsmittel wie beispielsweise Dimethylsulfoxid, Aceton, Acetonitril verwendet werden.

Üblicherweise wird die Biotransformation in Gegenwart einer C-Quelle durchgeführt. Als C-Quelle sind beispielsweise Kohlenhydrate wie Glucose, Fructose oder Saccharose und Zuckeralkohole wie Glycerin geeignet.

Der pH-Wert der Medien kann in einem Bereich von 5 bis 10, vorzugsweise von 6 bis 8, liegen.

Zweckmässig wird die Biotransformation bei einer Temperatur von 5 bis 60 °C, vorzugsweise von 10 bis 40 °C, durchgeführt.

Nach einer Umsetzungszeit von wenigen Minuten bis 50 h, kann dann das gewünschte Produkt in hoher Ausbeute und Enantiomerenreinheit (ee) isoliert werden.

### Beispiele

### Beispiel 1

### Anzucht der Mikroorganismen

Zellen von E. coli JNI109/pKAR,pKKGDH (DSMZ 11902) wurden in einem 20 1 Fermenter in 12 1 Mineralsalzmedium (Tabelle 1) bei 22 °C angezüchtet. Nach 6 h wurde IPTG hinzugegeben, um die Zellen zu induzieren. Dann wurde Glycerin hinzugegeben und die Zellen bis zu einer optischen Dichte OD₆₅₀ₙₘ = 41,8 innerhalb 52 h angezüchtet. Dann wurden die Zellen bei -80 °C aufbewahrt.

**Tabelle 1**

| | |
|---|---|
| Hefeextrakt | 0,5 g/l |
| Glycerin | 30 g/l |
| MgCl₂ x 6 H₂O | 0.8 g/l |
| CaCl₂ | 0,16 g/l |
| (NH₄)₂SO₄ | 2,0 g/l |
| SLF-Lösung | 1,0 ml/l |
| Fe-EDTA-Lösung | 1,5 ml/l |
| PPG-2000 | 0,1 g/l |
| Na₂HPO₄ x 2H₂O | 1,0 g/l |
| KH₂PO₄ | 1,0 g/l |
| K₂HPO₄ | 1,0 g/l |
| Thiamin | 10 mg/l |
| | |

| **SLF-Lösung:** | |
|---|---|
| KOH | 15,1 g/l |
| EDTA Na₂ x 2 H₂O | 100 g/l |
| ZnSO₄ x 7 H₂O | 9,0 g/l |
| MnCl₄ x 4H₂O | 4,0 g/l |
| H₃BO₃ | 2,7 g/l |
| CoCl₃ x 6H₂O | 1,8 g/l |
| CuCl₂ x 2H₂O | 1,5 g/l |
| NiCl₂ x 6H₂O | 0,18 g/l |
| Na₂MoO₄ x 2H₂O | 0,27 g/l |
| | |

| **Fe-EDTA-Lösung:** | |
|---|---|
| KOH | 10 g/L |
| EDTANa₂ x 2H₂O | 50 g/L |
| FeSO₄ x 7H₂O | 20 g/l |

### Beispiel 2

### Herstellung von 4,4,4-Trifluor-3(R)-hydroxybuttersäureethylester

a) Zu 800 ml Mineraisaizmedium (Tabelle 1) enthaltend E.coli JM109/ pKAR,pKKGDH bei einer OD₆₅₀ₙₘ von 7,2 wurden 140 g Glucose und 0,56 g NADPH hinzugegeben. 400 ml Butylacetat enthaltend 70 g 4,4,4-Trifluoracetoacetatethylester wurde hinzugefügt und die resultierende Mischung in einen 2 l Fermenter gegeben, bei 400 Upm gerührt und mit Luft (400 ml/min.) begast. Der pH wurde durch Zugabe von 1 M Na₂CO₃ auf 6,0 gehalten. Nach 24 h enthielt die organische Phase 48 g 4,4,4-Trifluor-3(R)-hydroxybuttersäureethylester mit einem ee-Wert von >99%, entsprechend einer molaren Ausbeute von 67,8 %.
b) Zu 800 ml Kaliumphosphatpuffer (100 mM, pH 6,0) enthaltend die Mikroorganismen gemäss Beispiel 1 bei einer OD₆₅₀ₙₘ von 30,7 wurden 140 g Glucose und 0,56 g NADPH hinzugefügt. 400 ml Butylacetat enthaltend 70 g 4,4,4-Trifluoracetoacetatethylester wurden hinzugegeben und die resultierende Mischung in einen Fermenter entsprechend Beispiel 2a eingespeist. Der pH wurde durch Zugabe von 1 M Na₂CO₃ auf pH 6,0 gehalten. Nach 25 h wurden nochmals 10 g 4,4,4-Trifluoracetoacetatethylester hinzugefügt. Nach 45 h enthielt die organische Phase 49 g 4,4,4-Trifluor-3(R)-hydroxybuttersäureethylester mit einem ee-Wert von > 99 %, entsprechend einer molaren Ausbeute von 60,6 %.
c) Zu 800 ml Kaliumphosphatpuffer (0,1 M, pH 6,0) enthaltend E. coli JM109/ pKAR,pKKGDH bei einer OD₆₅₀ von 7,6 wurden 140 g Glucose und 50 mg NADPH zugegeben. 400 ml Butylacetat enthaltend 70 g 4,4,4-Trifluoracetoacetatethylester wurden hinzugefügt und die resultierende Mischung in einen 2-1-Fermenter gegeben, mit 400 Upm gerührt und mit Luft (400 ml/min) begast. Der pH wurde durch Zugabe von 1M Na₂CO₃ auf 6.0 gehalten. Weitere 50 mg NADPH wurden 5 h nach Start des Fermenters zugegeben Nach 24 h enthielt die organische Phase 50 g 4,4,4-Trifluor-3(R)-hydroxybuttersäureethylester mit einem ee-Wert von >99,8%, entsprechend einer molaren Ausbeute von 71%.
d) Zu 800 ml Kaliumphosphatpuffer (0,1 M, pH 6,0) enthaltend E. coli DH5/pKAR, pKKGDH bei einer OD₆₅₀ von 6,5 wurden 140 g Glucose und 50mg NADPH zugegeben. 400 ml Butylacetat enthaltend 70 g 4,4,4-Trifluoracetoacetatethylester wurden hinzugefügt und die resultierende Mischung in einen 2-1-Fermenter gegeben, mit 400 Upm gerührt und mit Luft (400 ml/min) begast. Der pH wurde durch Zugabe von 1M Na₂CO₃ auf 6,0 gehalten. Weitere 50 mg NADPH wurden jeweils nach 5 h und 26 h zugegeben. Nach 46 h enthielt die organische Phase 35 g 4,4,4-Trifluor-3(R)-hydroxybuttersäureethylester mit einem ee-Wert von 99,7%, entsprechend einer molaren Ausbeute von 51%.

### Beispiel 3

### Herstellung von 4,4,4-Trifluor-3(R)-hydroxybuttersäureisopropylester

a) Zu 800 ml Mineralsalzmedium entsprechend Beispiel 1 enthaltend E. coli JM109/pKAR, pKKGDH bei einer OD₆₅₀ₙₘ von 9,7 wurden 140 g Glucose und 0,56 g NADPH hinzugefügt. 400 ml Butylacetat enthaltend 70 g 4,4,4-Trifluoressigsäureisopropylester wurden hinzugegeben und die resultierende Mischung in einem Fermenter entsprechend Beispiel 2 eingespeist. Der pH wurde durch Zugabe von 1 M Na₂CO₃ auf pH 6,0 gehalten. Nach 21' h enthielt die organische Phase 42,2 g (R)-4,4,4-Trifluor-3-hydroxybuttersäureisopropylester mit einem ee-Wert von >99 %, entsprechend einer molaren Ausbeute von 59,7%.
b) Zu 800 ml Kaliumphosphatpuffer (0, 1 M, pH 6,0) enthaltend E. coli DH5/pKAR,pKJKGDH bei einer OD₆₅₀ von 8,5 wurden 140 g Glucose und 50mg NADPH zugegeben. 400 ml Butylacetat enthaltend 70 g 4,4,4-Trifluoracetoacetatisopropylester wurden hinzugefügt und die resultierende Mischung in einen 2-1-Fermenter gegeben, mit 400 Upm gerührt und mit Luft (400 ml/min) begast. Der pH wurde durch Zugabe von 1M Na₂CO₃ auf 6,0 gehalten. Weitere 50 mg NADPH wurden 5 h nach Start des Fermenters zugegeben. Nach 24 h enthielt die organische Phase 32 g 4,4,4-Trifluor-3(R)-hydroxybuttersäureisopropylester mit einem ee-Wert von >99,9%, entsprechend einer molaren Ausbeute von 46%.

### Beispiel 4

### Herstellung von 4,4,4,-Trifluor-3(R)-hydroxybuttersäurehexylester

Zu 800 ml Kaliumphosphatpuffer (0,1 M, pH 6,0) enthaltend E. coli DH5/ pKAR,pKKGDH bei einer OD₆₅₀ von 9,5 wurden 140 g Glucose und 50 mg NADPH gegeben. 400 ml Butylacetat enthaltend 70 g 4,4,4-Trifluoracetoacetathexylester wurden hinzugefügt und die resultierende Mischung in einen 2-1-Fermenter gegeben, mit 400 Upm gerührt und mit Luft (400 ml/min) begast. Der pH wurde durch Zugabe von 1M Na₂CO₃ auf 6,0 gehalten. Weitere 50 mg NADPH wurden 5 h nach Start des Fermenters zugegeben. Nach 24 h enthielt die organische Phase 2g 4,4,4-Trifluor-3(R)-hydroxybuttersäurehexylester mit einem ee-Wert von >99,9%, entsprechend einer molaren Ausbeute von 3%.

### Beispiel 5

### Herstellung von 4,4,4,-Trifluor-3(R)-hydroxybuttersäurecyclohexylester

Zu 800 ml Kaliumphosphatpuffer (0,1 M, pH 6,0) enthaltend E. coli DH5/pKAR,pKKGDH bei einer OD₆₅₀ von 8,9 wurden 140 g Glucose und 50 mg NADPH zugegeben. 400 ml Butylacetat enthaltend 70 g 4,4,4-Trifluoracetoacetatcyclohexylester wurden hinzugefügt und die resultierende Mischung in einen 2-1-Fermenter gegeben, mit 400 Upm gerührt und mit Luft (400 ml/min) begast. Der pH wurde durch Zugabe von 1M Na₂CO₃ auf 6,0 gehalten. Weitere 50 mg NADP wurden 5 nach Start des Fermenters zugegeben. Nach 24 h enthielt die organische Phase 16 g 4,4,4-Trifluor-3(R)-hydroxybuttersäurecyclohexylester mit einem ee-Wert von >99.9%, entsprechend einer molaren Ausbeute von 23%.

### Beispiel 6

### Herstellung von 4,4,4,-Trifluor-3(R)-hydroxybuttersäurebenzylester

Zu 800 ml Kaliumphosphatpuffer (0,1 M, pH 6,0) enthaltend E. coli DH5/pKAR,pKKGDH bei einer OD₆₅₀ von 9,0 wurden 140 g Glucose und 50 mg NADPH zugegeben. 400 ml Butylacetat enthaltend 70 g 4,4,4-Trifluoracetoacetatbenzylester wurden hinzugefügt und die resultierende Mischung in einen 2-1-Fermenter gegeben, mit 400 Upm gerührt und mit Luft (400 ml/min) begast. Der pH wurde durch Zugabe von 1M Na₂CO₃ auf 6,0 gehalten. Weitere 50 mg NADPH wurden 5 h nach Start des Fermenters zugegeben. Nach 24 h enthielt die organische Phase 6 g 4,4,4-Trifluor-3(R)-hydroxybuttersäurebenzylester mit einem ee-Wert von >99,9%, entsprechend einer molaren Ausbeute von 9%.

### Beispiel 7

### Herstellung von 4,4,4,-Trifluor-3(R)-hydroxybuttersäure-2-ethoxyethylester

Zu 600 ml Kaliumphosphatpuffer (0,1 M, pH 6,0) enthaltend E. coli DH5/pKAR,pKKGDH bei einer OD₆₅₀ von 10,2 wurden 105 g Glucose und 37,5mg NADPH zugegeben. 300 ml Butylacetat enthaltend 35 g 4,4,4- Trifluoracetoacetatethoxyethylester wurden hinzugefügt und die resultierende Mischung in einen 2-1-Fermenter gegeben, mit 400 Upm gerührt und mit Luft (300 ml/min) begast. Der pH wurde durch Zugabe von 1M Na₂CO₃ auf 6,0 gehalten. Weitere 37,5 mg NADPH wurden 5 h nach Start des Fermenters zugegeben. Nach 24 h enthielt die organische Phase 4 g 4,4,4-Trifluor-3(R)-hydroxybuttersäureethoxyethylester mit einem ee-Wert von 98,6%, entsprechend einer molaren Ausbeute von 12%.

### Beispiel 8

### Herstellung von 4,4,4,-Trifluor-3(R)-hydroxybuttersäure-2-(2-ethoxyethoxy)ethylester

Zu 600 ml Kaliumphosphatpuffer (0.1 M, pH 6,0) enthaltend E. coli DH5/pKAR,pKKGDH bei einer OD₆₅₀ von 10,7 wurden 105 g Glucose und 37,5 mg NADPH zugegeben. 300 ml Butylacetat enthaltend 35 g 4,4,4-Trifluoracetoacetatethoxyethoxyethylester wurden hinzugefügt und die resultierende Mischung in einen 2-1-Fermenter gegeben, mit 400 Upm gerührt und mit Luft (300 ml/min) begast. Der pH wurde durch Zugabe von 1M Na₂CO₃ auf 6,0 gehalten. Weitere 37,5 mg NADPH wurden 5 h nach Start des Fermenters zugegeben. Nach 24 h enthielt die organische Phase 5 g 4,4,4-Trifluor-3(R)-hydroxybuttersäureethoxyethoxyethylester mit einem ee-Wert von >99,9%, entsprechend einer molaren Ausbeute von 16%.

### Beispiel 9

### Herstellung von 4,4,4,-Trifluor-3(R)-hydroxybuttersäuremethylester

Zu 600 ml Kaliumphosphatpuffer (0,1 M, pH 6,0) enthaltend E. coli DH5/pKAR,pKKGDH bei einer OD₆₅₀ von 11,4 wurden 105 g Glucose und 37,5mg NADPH zugegeben. 300 ml Butylacetat enthaltend 33 g 4,4,4-Trifluoracetoacetatmethylester wurden hinzugefügt und die resultierende Mischung in einen 2-1-Fermenter gegeben, mit 400 Upm gerührt und mit Luft (300 ml/min) begast. Der pH wurde durch Zugabe von 1M Na₂CO₃ auf 6,0 gehalten. Weitere 37,5 mg NADPH wurden 5 h nach Start des Fermenters zugegeben. Nach 24 h enthielt die organische Phase 3,6 g 4,4,4-Trifluor-3(R)-hydroxybuttersäuremethylester mit einem ee-Wert von 96.1%, entsprechend einer molaren Ausbeute von 7%.

## Patentansprüche

1. Verfahren zur Herstellung von 4,4,4-Trifluoro-3(R)-hydroxybuttersäurederivaten der allgemeinen Formel 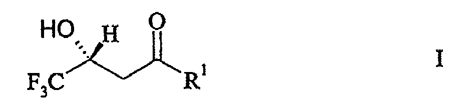 worin
R¹ = -OR², worin R² Wasserstoff, C₁₋₁₀-Alkyl, gesättigt oder ungesättigt, C₃₋₈Cycloalkyl, Aryl, Alkoxyalkyl oder Alkoxyalkoxyalkyl ist, oder
-NR³R⁴, worin R³ und R⁴ gleich oder verschieden sind und für Wasserstoff, C₁₋₁₀-Alkyl, C₁₋₁₀-Alkenyl, C₃₋₈-Cycloalkyl oder Aryl stehen, oder
-SR⁵, worin R⁵ Wasserstoff, C₁₋₁₀-Alkyl, C₁₋₁₀-Alkenyl, Aryl oder C₃₋₈-Cycloalkyl ist,
umfassend die Umsetzung eines Trifluoracetessigsäurederivats der allgemeinen Formel 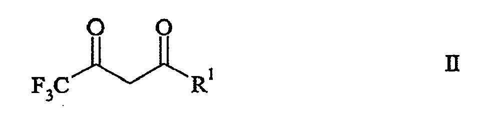 worin R¹ die genannte Bedeutung hat, mit einem ee-Wert >99% mittels Mikroorganismen der Gattung *Escherichia,* die zur Expression mit einem für eine NADPH-abhängige Aldehydreductase aus *Sporobolomyces* codierendem Gen transformiert sind, und die ein Gen codierend für eine Dehydrogenase exprimieren, welche Dehydrogenase als NADPH-Generator die Bildung von NADPH aus dessen oxidierter Form NADPH katalysiert, oder mittels einem zellfreien Enzymextrakt dieser Mikroorganismen.

2. Verfahren nach Anspruch 1, **dadurch gekennzeichnet, dass** man die Umsetzung mittels Mikroorganismen der Spezies *Escherichia coli,* transformiert mit einem Gen, codierend für die Dehydrogenase, durchführt.

3. Verfahren nach Anspruch 2, **dadurch gekennzeichnet, dass** die Dehydrogenase eine Glucosedehydrogenase ist.

4. Verfahren nach Anspruch 3, **dadurch gekennzeichnet, dass** die Glucosedehydrogenase die Glucosedehydrogenase aus *Bacillus megaterium* ist.

5. Verfahren nach Anspruch 1, **dadurch gekennzeichnet, dass** man die Umsetzung mittels Mikroorganismen der Spezies *Escherichia coli JM109, Escherichia coli HB 101* oder *Escherichia coli DH5* durchführt.

6. Verfahren nach einem der Ansprüche 1 bis 5, **dadurch gekennzeichnet, dass** die NADPH-abhängige Aldehydreductase aus *Sporobolomyces salmonicolor* ist.

7. Verfahren nach Anspruch 6, **dadurch gekennzeichnet, dass** die NADPH-abhängige Aldehydreductase die Aldehydreductase aus *Sporobolomyces salmonicolor* ist wie hinterlegt mit dem Plasmid pKAR unter der Hinterlegungsnummer DSM11902.

8. Verfahren nach mindestens einem der Ansprüche 2 bis 5, **dadurch gekennzeichnet, dass** man die Umsetzung durchführt mittels den mit den Plasmiden pKAR und PKKGDH transformieren Mikroorganismen *Escherichia coli JM109* wie hinterlegt unter der Hinterlegungsnummer DSM11902 oder *Escherichia coli DH5* wie hinterlegt unter der Hinterlegungsnummer DSM12566, welche Plasmide zur Expression sowohl für eine NADPH-abhängige Aldehydreductase, welche befähigt ist, eine Carbonylfunktion zu reduzieren als auch für eine die Bildung von NADPH katalyiserenden Glucosedehydrogenase, codieren.

9. Verfahren nach mindestens einem der vorhergehenden Ansprüche, **dadurch gekennzeichnet, dass** man die Biotransformation bei einer Temperatur von 5 bis 60 °C durchgeführt.

10. Verfahren nach Anspruch 9, **dadurch gekennzeichnet, dass** man die Biotransformation bei einem pH Wert von 5 bis 10 durchführt.

11. Rekombinanter Mikroorganismus der Gattung *Escherichia* zur Durchführung des Verfahrens nach Anspruch 1, der zur Expression mit einem für eine NADPH-abhängige Aldehydreductase aus *Sporobolomyces* codierendem Gen transformiert ist, und der mit einem Gen codierend für eine Dehydrogenase transformiert ist und diese Dehydrogenase exprimiert, welche Dehydrogenase als NADPH-Generator die Bildung von NADPH aus dessen oxidierter Form NADPH katalysiert.

12. Mikroorganismus nach Anspruch 11, **dadurch gekennzeichnet, dass** der Mikroorganismus ein Mikroorganismus der Spezies *Escherichia coli* ist.

13. Mikroorganismus nach Anspruch 11 oder 12, **dadurch gekennzeichnet, dass** die Dehydrogenase eine Glucosedehydrogenase ist.

14. Mikroorganismus nach Anspruch 3, **dadurch gekennzeichnet, dass** die Glucosedehydrogenase die Glucosedehydrogenase aus *Bacillus megaterium* ist.

15. Mikroorganismus nach einem der Ansprüche 11 bis 14, **dadurch gekennzeichnet, dass** die NADPH-abhängige Aldehydreductase die Aldehydreductase aus *Sporobolomyces salmonicolor* ist wie hinterlegt mit dem Plasmid pKAR unter der Hinterlegungsnummer DSM11902.

16. Mikroorganismus nach Anspruch 15, **dadurch gekennzeichnet, dass** der Mikroorganismus der Stamm *Escherichia coli JM109* wie hinterlegt unter der Hinterlegungsnummer DSM11902 oder *Escherichia coli DH5* wie hinterlegt unter der Hinterlegungsnummer DSM 12566 ist.

## Claims

1. Process for preparing 4,4,4-trifluoro-3(R)-hydroxybutyric acid derivatives of the general formula 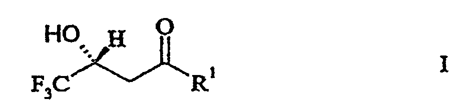 where
R¹ is -OR², where R² is hydrogen, C₁₋₁₀-alkyl, saturated or unsaturated, C₃₋₈-cycloalkyl, aryl, alkoxyalkyl or alkoxyalkoxyalkyl, or
-NR³R⁴, where R³ and R⁴ are identical or different and represent hydrogen, C₁₋₁₀-alkyl, C₁₋₁₀-alkenyl, C₃₋₈-cycloalkyl or aryl, or
-SR⁵, where R⁵ is hydrogen, C₁₋₁₀-alkyl, C₁₋₁₀-alkenyl, aryl or C₃₋₈-cycloalkyl,
comprising the conversion of a trifluoroacetic acid derivative of the general formula 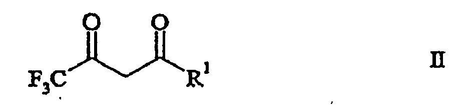 where R¹ is as defined above, with an ee value >99% by means of microorganisms of the genus *Escherichia* which, for expression, have been transformed with a gene coding for an NADPH-dependent aldehyde reductase from *Sporobolomyces,* and which express a gene coding for a dehydrogenase which, as an NADPH generator, catalyses the formation of NADPH from its oxidized form NADPH, or by means of a cell-free enzyme extract of these microorganisms.

2. Process according to Claim 1, **characterized in that** the conversion is carried out by means of microorganisms of the species *Escherichia coli* transformed with a gene coding for the dehydrogenase.

3. Process according to Claim 2, **characterized in that** the dehydrogenase is a glucose dehydrogenase.

4. Process according to Claim 3, **characterized in that** the glucose dehydrogenase is the glucose dehydrogenase from *Bacillus megaterium.*

5. Process according to Claim 1, **characterized in that** the conversion is carried out by means of microorganisms of the species *Escherichia coli JM109, Escherichia coli HB 101* or *Escherichia coli DH5.*

6. Process according to any one of Claims 1 to 5, **characterized in that** the NADPH-dependent aldehyde reductase is from *Sporobolomyces salmonicolor.*

7. Process according to Claim 6, **characterized in that** the NADPH-dependent aldehyde reductase is the aldehyde reductase from *Sporobolomyces salmonicolor* as deposited with the plasmid pKAR under the deposition number DSM11902.

8. Process according to at least one of Claims 2 to 5, **characterized in that** the conversion is carried out by means of the microorganisms *Escherichia coli JM109* as deposited under the deposition number DSM11902, or *Escherichia coli DH5,* as deposited under the deposition number DSM12566, these microorganisms having been transformed with the plasmids pKAR and PKKGDH, which plasmids code, for expression, not only for an NADPH-dependent aldehyde reductase capable of reducing a carbonyl function but also for a glucose dehydrogenase which catalyses the formation of NADPH.

9. Process according to at least one of the preceding claims, **characterized in that** the biotransformation is carried out at a temperature of 5 to 60°C.

10. Process according to Claim 9, **characterized in that** the biotransformation is carried out at a pH of 5 to 10.

11. Recombinant microorganism of the genus *Escherichia* for carrying out the process according to Claim 1, which, for expression, has been transformed with a gene coding for an NADPH-dependent aldehyde reductase from *Sporobolomyces* and which has been transformed with a gene coding for a dehydrogenase and expresses this dehydrogenase, which dehydrogenase, as an NADPH generator, catalyses the formation of NADPH from its oxidized form NADPH.

12. Microorganism according to Claim 11, **characterized in that** the microorganism is a microorganism of the species *Escherichia coli.*

13. Microorganism according to Claim 11 or 12, **characterized in that** the dehydrogenase is a glucose dehydrogenase.

14. Microorganism according to Claim 13, **characterized in that** the glucose dehydrogenase is the glucose dehydrogenase from *Bacillus megaterium.*

15. Microorganism according to any one of Claims 11 to 14, **characterized in that** the NADPH-dependent aldehyde reductase is the aldehyde reductase from *Sporobolomyces salmonicolor* as deposited with the plasmid pKAR under the deposition number DSM11902.

16. Microorganism according to Claim 15, **characterized in that** the microorganism is the strain *Escherichia coli JM109* as deposited under the deposition number DSM11902 or *Escherichia coli DH5* as deposited under the deposition number DSM12566.

## Revendications

1. Procédé de préparation de dérivés d'acide 4,4,4-trifluoro-3(R)-hydroxybutyrique de formule générale : 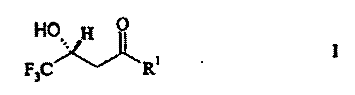 dans laquelle
R¹ = -OR² dans laquelle R² représente l'hydrogène, un alkyle en C₁ à C₁₀, saturé ou insaturé, un cycloalkyle en C₃ à C₈, un aryle, un alcoxyalkyle ou un alcoxyalcoxyalkyle ou
-NR³R⁴, dans laquelle R³ et R⁴ sont identiques ou différents et représentent l'hydrogène, un alkyle en C₁ à C₁₀, un alcényle en C₁ à C₁₀, un cycloalkyle en C₃ à C₈ ou un aryle ou
-SR⁵, dans laquelle R⁵ représente l'hydrogène, un alkyle en C₁ à C₁₀, un alcényle en C₁ à C₁₀, un aryle ou un cycloalkyle en C₃ à C₈,
lequel procédé comprend la conversion d'un dérivé d'acide trifluoroacétique de formule générale : 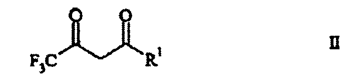 dans laquelle
R¹ a la signification donnée plus haut, avec un indice ee > 99 %, au moyen de microorganismes de type *Escherichia* qui sont transformés avec un gène qui code pour une aldéhyde réductase de *Sporobolomyces* qui agit sous la dépendance du NADPH et qui exprime un gène qui code pour une déshydrogénase, laquelle déshydrogénase catalyse en tant que créatrice de NADPH la formation de NADPH à partir de sa forme oxydée NADPH, ou au moyen d'un extrait d'enzyme exempt de cellules de ces microorganismes.

2. Procédé selon la revendication 1, **caractérisé en ce que** la conversion est réalisée au moyen de microorganismes du type *Escherichia coli* transformés avec un gène qui code pour la déshydrogénase.

3. Procédé selon la revendication 2, **caractérisé en ce que** la déshydrogénase est une glucose déshydrogénase.

4. Procédé selon la revendication 3, **caractérisé en ce que** la glucose déshydrogénase est la glucose déshydrogénase de *Bacillus megaterium.*

5. Procédé selon la revendication 1, **caractérisé en ce que** la conversion est réalisée au moyen de microorganismes des types *Escherichia coli JM 109, Escherichia coli HB101* ou *Escherichia coli DH5.*

6. Procédé selon l'une des revendications 1 à 5, **caractérisé en ce que** l'aldéhyde réductase qui dépend de NADPH est l'aldéhyde réductase de *Sporobolomyces salmonicolor.*

7. Procédé selon la revendication 6, **caractérisé en ce que** l'aldéhyde réductase qui dépend de NADPH est l'aldéhyde réductase de *Sporobolomyces salmonicolor* déposé avec le plasmide pKAR sous le numéro de dépôt DSM11902.

8. Procédé selon au moins l'une des revendications 2 à 5, **caractérisé en ce que** la conversion est réalisée au moyen des microorganismes *Escherichia coli JM109* transformés avec le plasmide pKAR et PKKGDH et déposés sous le numéro de dépôt DSM11902 ou *Escherichia coli DH5* déposés sous le numéro de dépôt DSM12566, lequel plasmide code pour l'expression à la fois d'une aldéhyde réductase dépendante du NADPH et apte à réduire une fonction carbonyle ainsi que pour une glucose déshydrogénase qui catalyse la formation de NADPH.

9. Procédé selon au moins l'une des revendications précédentes, **caractérisé en ce que** la biotransformation est réalisée à une température de 5 à 60°C.

10. Procédé selon la revendication 9, **caractérisé en ce que** la biotransformation est réalisée à un pH d'une valeur comprise entre 5 et 10.

11. Microorganisme recombiné du type *Escherichia,* destiné à mettre en oeuvre le procédé selon la revendication 1 et transformé en vue de l'expression par un gène qui code pour une aldéhyde réductase de *Sporobolomyces* dépendant de NADPH, transformée avec un gène qui code pour une déshydrogénase et exprimant cette déshydrogénase, laquelle déshydrogénase servant de créatrice de NADPH en catalysant la formation de NADPH à partir de sa forme oxydée NADPH.

12. Microorganisme selon la revendication 11, **caractérisé en ce que** le microorganisme est un microorganisme de type *Escherichia coli.*

13. Microorganisme selon les revendications 11 ou 12, **caractérisé en ce que** la déshydrogénase est une glucose déshydrogénase.

14. Microorganisme selon la revendication 13, **caractérisé en ce que** la glucose déshydrogénase est la glucose déshydrogénase de *Bacillus megaterium.*

15. Microorganisme selon l'une des revendications 11 à 14, **caractérisé en ce que** l'aldéhyde réductase dépendant de NADPH est l'aldéhyde réductase de *Sporobolomyces salmonicolor* déposé avec le plasmide pKAR sous le numéro de dépôt DSM11902.

16. Microorganisme selon la revendication 15, **caractérisé en ce que** le microorganisme est le microorganisme de la souche *Escherichia coli JM109* déposée sous le numéro de dépôt DSM11902 ou *Escherichia coli DH5* déposée sous le numéro de dépôt DSM12566.
